# EUROPEAN PATENT SPECIFICATION

(11) **EP 0 721 445 B1**
(45) Date of publication and mention of the grant of the patent: **15.12.1999**
(21) Application number: 94929124.9
(22) Date of filing: 02.09.1994
(51) Int. Cl.: C07C 31/30

(54) **IMPROVED METHOD OF PREPARATION OF LITHIUM TERTIARY-BUTOXIDE**
VERBESSERTES VERFAHREN ZUR HERSTELLUNG VON LITHIUM-TERTIÄR-BUTOXID
PROCEDE AMELIORE DE PREPARATION DE BUTOXYDE TERTIAIRE DE LITHIUM

(30) Priority: 30.09.1993 US 129818
(43) Date of publication of application: 17.07.1996
(73) Proprietor: FMC CORPORATION, Philadelphia, PA 19103 (US)
(72) Inventor: MORRISON, Robert, Charles, Gastonia, NC 28056 (US); KAMIENSKI, Conrad, William, Gastonia, NC 28054 (US); SCHWINDEMAN, James, Anthony, Charlotte, NC 28269 (US)
(74) Representative: W.P. THOMPSON & CO.
(86) International application number: US9409844
(87) International publication number: WO9509141

(56) References cited:
- US-A- 3 971 833
- COLLECTION CZECHOSLOV. CHEM. COMMUN., Vol. 35, issued 1970, LOCHMAN et al., "Preparation of some alkoxides of alkaline metals", pages 733-736.
- W. NOVIS SMITH, JR.: "PREPARATION OF TERT-BUTYLLITHIUM", J.ORGANOMET.CHEM., 27-06-1994, vol. 82, pages 1 to 6

## Description

This invention concerns a process for preparing lithium tertiary alkoxides by reacting lithium metal in gross or bulk form with a tertiary alcohol.

Alkali metal alkoxides and especially alkali metal tertiary alkoxides such as lithium tertiary butoxide are used in the preparation of pharmaceutical intermediates and in the synthesis of poiymers and in general organic synthesis. U.S. Patent 5,276,219 issued January 4, 1994 describes a process for preparing clear, suspensoid-free solutions of lithium tert-butoxide in tetrahydrofuran at reflux by reaction of lithium metal in finely divided dispersed particle form with an excess of tert-butyl alcohol.

U.S. Patent No. 3,971,833 is directed to a process for making alkali or alkaline earth metal alcoholate in which an alkali or alkaline earth metal is dissolved or liquified in an inert solvent and contacted with an alcohol under conditions of elevated pressure and temperature. Similarly, collection Czechoslov. Chem. Commun; Vol 35, 1970, Lochman et al "Preparation of some alkoxides of alkaline metals", pages 733-736 teaches processes for making alkoxides of alkaline metals under conditions to liquify the metal during the reaction.

Although lithium metal in a finely divided dispersed state reacts rapidly with tert-butyl alcohol in THF medium, it is costly to produce, requiring the steps of (a) heating of bulk lithium metal and mineral oil to about 190-200°C in the presence of a dispersion aid, such as oleic acid, (b) stirring the resultant molten mixture at high speeds in a special dispersion unit to produce the required small particle sizes (generally less than 100 microns), then (c) cooling the product (preferably without stirring), and then finally (d) removing the mineral oil from the solidified lithium metal particles by washing several times with a volatile hydrocarbon solvent, such as hexane or pentane. The volatile hydrocarbon solvent can optionally be removed by purging with an inert gas such as argon, or more preferably, be washed one or more times with tetrahydrofuran before reaction with the tert-butyl alcohol.

Besides being costly to produce, lithium dispersion also may add undesirable impurities to the subsequent lithium tert-butoxide product in THF such as, e.g., mineral oil and oleic acid breakdown products, and volatile hydrocarbons.

Because of the extremely small sizes of the lithium metal particles, a high proportion of solid impurities, small in size may also be present after reaction with the alcohol is complete. Impurities on the lithium surface slow down the initial reaction. These impurities arise from side reactions with traces of oxygen in the inert atmosphere, of traces of water in the solvent and alcohol, and with the solvent itself. These small solid impurities (including any unreacted lithium metal particles) cause filtration problems, although the use of an excess of the alcohol generally takes care of any unreacted lithium metal.

The present invention provides a process for quickly preparing easily separable solutions of lithium tert-alkoxides in an economically feasible time period (1 to 10 hours), comprising the steps of: reacting lithium bulk metal whose size exceeds 0.1 mm, containing less than 0.6% by weight of sodium, with a tertiary alkyl alcohol containing 3 to 10 carbon atoms in mole ratios of lithium metal to alcohol ranging from 1 to 1 to 10 to 1 in an ethereal or hydrocarbon medium under an inert atmosphere at an elevated temperature between 40°C and 100°C, cooling the product and separating the product solution from the unreacted lithium metal in the reactor; the process is conveniently continued by adding additional solvent, sufficient lithium metal and alcohol to the unreacted metal in the reactor to maintain the mole ratio of lithium metal to alcohol, and continuing the reaction, thereby to form further lithium tert-alkoxide, and repeating said steps a number of times. Optionally the reaction may be catalyzed with small amounts of a C₁ to C₃ alcohol. Lithium bulk metal and lithium in bulk form are synonymous terminology as used in the description of this invention.

Unexpectedly, the process of the present invention overcomes problems experienced with the use of lithium metal in a finely divided (dispersed) state without experiencing an overly great increase in reaction time by the use of a sufficient excess of lithium metal in bulk form (over and above the alcohol used) in the essential absence of any hydrocarbon or other (solid) impurities. Impurities on the lithium surface slow down the initial reaction. Surprisingly, the amount of lithium metal in bulk form needed to preserve a comparable overall reaction time when compared to lithium in dispersed form is only in the order of about three times. For example, the surface area of one gram equivalent of lithium metal particles 20 microns in diameter is about 13,000 square centimeters, while the surface area of an equivalent amount of lithium metal cubes with dimensions of one centimeter by one centimeter by one centimeter is only 79 square centimeters, a one hundred sixty-six times greater surface area for the dispersed lithium. One would therefore expect the relative amount of lithium in bulk form needed to give a overall reaction rate comparable to the dispersed lithium to be in the order of one hundred sixty-six times greater. Instead, only three equivalents of lithium metal in the form of cubes was found to react with one equivalent of the alcohol in a comparable overall time (180 minutes) as did one equivalent of lithium metal dispersed to 20 micron diameter particles (135 minute overall reaction time). See Table I. Lithium tertiary butoxide in the tables I-V is abbreviated as LTB and elsewhere as lithium tert-butoxide and lithium t-butoxide.

After the reaction of one of the three equivalents of the bulk lithium metal is completed, the slightly hazy product solution can be easily decanted from the unreacted (floating) lithium metal and filtered quickly. Another reaction can be started in the same reactor, adding only another equivalent each of lithium metal and alcohol and the required amount of solvent. When the process is to be repeated one or more times in the same reactor it is advantageous to immediately add additional solvent after the product of the preceding reaction is recovered. Surprisingly, the overall reaction time decreases still further in the second run (130 minutes) and then stays constant for the next two consecutive runs (120 minutes). This decrease in time is thought to be due to preconditioning of the lithium metal. (Removal of surface impurities).

The ease of separation of the product solution from unreacted lithium metal is maintained in each subsequent run and there is no hazardous unreacted lithium to be disposed of as in the runs using dispersed lithium.

It is known that elevated sodium levels in the lithium metal used in preparing alkyllithiums are beneficial. For example W.N. Smith, Jr., in Journal of Organometallic Chemistry, 82 (1974) 1-6 recommends the use of 1% by weight of sodium in the lithium metal used to prepare tertiary butyllithium from tert-butyl chloride. It is also stated that lower levels of sodium (0.02%) in the lithium metal resulted in no reaction with the alkyl chloride. It has unexpectedly been determined that at a sodium level of 0.69 weight percent sodium (in the lithium cubes) the overall reaction time is about 6 to 7 hours, whereas at a sodium level of 0.002%, the reaction time is 1.5 hours, everything else being equal.

In the case of dispersed lithium containing 0.79 weight percent sodium, the overall reaction rate is six hours as compared to a three hour reaction time for lithium dispersion containing 0.002 percent sodium.

In another embodiment of the invention, certain catalysts are used to speed up the overall reaction time of bulk lithium metal pieces with tert-butyl alcohol in THF solution. With a mole ratio of t-butyl alcohol to lithium metal of 1.1, addition of small amounts (2-5 mole % based on tert-butyl alcohol) of C1 to C3 alcohols decreases the reaction time by about one-half when using lithium metal rod pieces (1.27 centimeter diameter by one centimeter long). The ratio of lithium metal to tert alcohol can be 0.9 to 1 to 10 to 1.

It is quite unexpected that the size of the metal pieces used in the reaction with tert-butyl alcohol in tetrahydrofuran can be varied widely, depending on the batch size of the runs, without unduly affecting [reducing] the overall reaction time. Thus, any of the common commercially available sizes of lithium metal may be employed or segments [pieces] may be cut from these. For example, in a forty gallon size run to prepare 184 moles of product, ten one inch thick trapezoidal-shaped pieces of lithium were cut from each of four two pound trapezoidal ingots having dimensions of 2.5/3.5 inches (6.4/8.9cm) in width by 3.25 inches (8.3cm) in height by 10.5 inches (26.7cm) in length, and charged to the reactor, along with the requisite amount of tert-butyl alcohol (mole ratio Li/ROH=3) and THF solvent. The mixture was heated to reflux and required 4-5 hours to completion [average conversion of alcohol of 99+ % over seven consecutive runs]. This overall reaction time compares favorably to one mole laboratory runs using much smaller pieces of metal [see Table VI, P.P. Series 6].

Sizes of lithium metal available commercially are: one inch (2.5cm) diameter by 8 inch (20.3cm) long rods, one-half inch 1.3cm) by 6.5 inch (16.5cm) long rods, 2.25 inch (5.7cm) by 3.38 inch (8.6cm) long cylindrical ingots [1/4 lb], 3 inch (7.6cm) diameter by 3.8 inch (9.7cm) long cylindrical ingots [1/2 lb (227g)], 4 inch (10.2cm) diameter by 5 (12.7cm) inch long cylindrical ingots [1 lb (454g)], and the two pound (908g) trapezoidal ingots mentioned above, as well as lithium metal dispersion, 30 weight per cent in mineral oil with 90% of the particles greater than 10 microns, but less than 50 microns, (average: 20 microns).

The optimum size of the lithium bulk metal employed will depend upon the size of the reaction being carried out. Generally, the size of the pieces of lithium being employed will be such that the overall reaction time will be less than 8-10 hours. The pieces of metal should be easily visible as being discrete particles, i.e., not particles produced by a dispersion process as described above [less than 0.1 millimeter].

Table I compares the sizes of some lithium metal pieces and their surface area per gram against the overall reaction time, including the feed time of the alcohol, and the run size. Note the longer overall reaction time for lithium dispersion versus bulk lithium [same size run].

Generally, it is preferable to use excesses of such bulk metal relative to the tert-butyl alcohol in the range of 2:1 to 10:1, but, most preferably, 3:1 to 5:1.

Feeding the tert-butyl alcohol gradually rather than adding it all to the reactor initially lengthens the reaction time somewhat, but not inordinately so. Essentially, the alcohol reacts about as quickly as it is added after the first run of a consecutive series is completed. It is thought that this first run of the series serves to condition the metal and acts much like the catalyst activation. Thus, the second run of such a series, where all of the alcohol is added at once, is completed in a period of 130 minutes (see Table III series I), whereas, in a second run where the alcohol is added gradually over a period of 108 minutes, the reaction is complete in 155 minutes [see Table III, Series 2].

The reactions may be carried out at ordinary [atmospheric] pressure, but the atmospheric composition above the contents of the reaction vessel should be inert. Thus, the atmosphere should be dry and inert to lithium metal, i.e., most favorably be argon gas. Higher pressures, i.e., those above atmospheric, may be employed to raise the reaction temperature and thus, to further speed up the reaction.

Regardless of which size lithium pieces one employs, it is preferable to use a low sodium containing grade of lithium metal. Preferably, one should use a grade of lithium metal containing 0.001 to 0.6% by weight of sodium based on lithium; more preferable is a sodium level of less than 0.1%, and most preferable is a sodium level of 0.001 to 0.01 weight percent of the lithium metal. For example, employing lithium dispersion at a 1:1 level with tert-butyl alcohol in THF medium, at a sodium level of 0.002 wt%,. the reaction is essentially completed (>95%) in two to three hours time, whereas if the sodium level of the lithium metal used is 0.79 wt%, the reaction, to achieve above 90% completion, requires an additional 3-4 hours. The lithium metal in the latter case agglomerates to give larger pieces of less reactive lithium. At intermediate levels of sodium [0.19% and 0.38%] the reactions proceed more readily and are also completed in 2-3 hours [see Table II].

In the case of bulk metal, this effect is also evident. Tables III and IV present data at low (<0.01 wt%) and high (>0.6 wt%) sodium levels in the lithium metal employed. At the lower sodium level, overall reaction times are of the order of 1.5 to 3 hours and the reactions are complete, whereas at the higher sodium level, this reaction time is 6 to 7 hours and the reactions are incomplete. Less soluble surface impurities appear to coat the metal in the later case and slow down the rate of reaction, especially near the end.

Catalysts are employed to speed up the overall rate of the reaction. These are generally lower molecular weight C₁ to C₃ alcohols, particularly methanol, ethanol, and isopropanol. Catalyst levels should be in the range of 2-10 mole% based on tert-butyl alcohol reactant, and most preferably, 3-7 mole%.

Table V presents results using these catalysts in runs with lithium metal rod pieces (1.27 cm diameter by 0.5 cm long). The overall reaction times are cut from 332 minutes (5.5 hours) to 180 to 218 minutes (3-3.6 hours) using 4-5 mole% of catalysts methanol, ethanol or isopropanol. Other higher alcohols (C₄ and greater) may be used, but do not generally give as good results.

Alcohol reactants other than tert-butyl alcohol can be employed, such as, for example, C₄-C₁₀ tertiary alcohols, such as, e.g., tert-amyl alcohol, 3-methyl-3-hexanol, 3-ethyl-3-pentanol, and 4-methyl-4-heptanol.

Solvents which may be used in the process of this invention to prepare lithium tert alkoxides are ethers such as ethyl ether, 1,2-dimethoxyethane, tetrahydrofuran, methyltetrahydrofuran, tetrahydropyran, and the like, and hydrocarbon solvents such as pentane, hexane, heptane, and toluene, and mixtures thereof. Although the most favored reaction temperature is the reflux temperature of the selected solvent, however, the reaction may be run below reflux, e.g., 40 to 100°C depending on the boiling point of the solvent. However, the least favorable temperatures are below about 40-45°C.

The reaction concentration of the product alkoxide being formed is generally limited by the solubility of the product in the solvent being employed. For example, the solubility of lithium tert-butoxide in tetrahydrofuran is approximately 2.4 moles per liter of solution. The preferred reaction concentration, however, is somewhat less than this, i.e., about 2.0 moles per liter of solution. Thus, it is best to carry out these reactions at concentration levels as high as possible without at the same time fostering a condition of insolubility of the product.

The following examples further illustrate the invention.

### Exemplary Runs of Lithium tert-Butoxide Employing Bulk Lithium [Table III, Series 1]

A volume of 426 milliliters of dry tetrahydrofuran and 76.71 grams [1.03 moles] of dry tert-butyl alcohol were added to a one liter three necked flask equipped with a mechanical stirrer, reflux condenser, thermometer, and gas inlet tube for provision of an argon atmosphere. The solution was heated to 61 degrees Centigrade and 24 pieces of lithium metal, cut into cubes with the approximate dimensions of one centimeter on each side [21.53 grams, 3.10 gram equivalents], added to the flask as rapidly as possible. Heating was continued to bring the mixture to reflux with stirring. After 30 minutes, a sample of the solution was taken [ 4.24 grams] and analyzed for alkalinity content and extent of conversion of the alcohol present in the reactor to product lithium alkoxide. Conversion at this point was 17.4%. Samples were taken at 60, 120, 150 and 180 minutes. Conversions were 37.6%, 84.4%, 92.6%, and 98.4%, respectively. After the 150 minute sample the metal pieces started losing their blue color and became shiny. At 180 minutes hydrogen evolution ceased and the heating was stopped. The shiny pieces were pitted. After the reaction mixture had cooled to room temperature, the slightly cloudy solution was decanted away from the unreacted excess lithium metal pieces and stored in an argon-flushed bottle. A weight of 7.9 grams of tert-butyl alcohol [0.1068 moles] was added to the bottle. To the remaining pieces of lithium metal in the reaction flask was added 439 milliliters of tetrahydrofuran. After standing overnight the solution contents of the bottle were clear.

### Second consecutive run:

To the above reactor, containing lithium and tetrahydrofuran, was added another eight pieces of lithium metal in the form of 1x1x1 centimeter [approximately] cubes [7.41 grams, 1.068 gram equivalents]. The reactor contents were heated to about 59°C and a solution of 79.1 grams [1.069 moles] of tert-butyl alcohol and 50 milliliters of tetrahydrofuran added all at once. The mixture was stirred and heated to reflux and, once again, samples removed to determine the extent of conversion at definite time periods. At 20, 40, 100, and 130 minutes the conversions were 23%, 38%, 90%, and 99%, respectively. After cooling the reaction mass to room temperature the cloudy solution was decanted away from the remaining lithium metal into an argon-flushed bottle and 7.8 grams [0.1054 moles] of tert-butyl alcohol added to the bottle. A volume of 416 milliliters of tetrahydrofuran was added to the reactor containing the remaining lithium metal. After standing overnight the solution contents of the bottle were clear.

### Third consecutive run:

Another eight pieces of lithium metal [7.02 grams] in the form of cubes was added to the above reactor from the second consecutive run, the mix heated to 53.7°C, 75.42 grams of t-butyl alcohol added and the reaction continued at reflux. Samples taken at 20, 60, 90, and 124 minutes showed conversions of 20, 52, 82, and 100%, respectively. The product solution was decanted away from the metal as described above, and 432 ml of THF added to the remaining metal in the reactor.

### Fourth consecutive run:

Eight pieces, 7.29 grams of lithium were added to the reactor, the mix heated to 51.3°C, 77.8 grams of t-butyl alcohol added, and the reaction carried on at reflux. Conversions at 20, 40, 80, and 120 min were 26, 37, 72, and 100%, resp. The product solution was decanted away and 434 ml THF added to the reactor. The product solution was then filtered and a clear solution obtained with a filtration time of four minutes. The solids on the filter plate were washed with 104.2 grams of a solution of seven grams of t-butyl alcohol in tetrahydrofuran and dissolved completely indicating that they were undissolved lithium t-butoxide. Analysis of both clear solutions [main product and wash] showed recovered yields of 90.5% and 6.2%, resp. for a total yield of 96.7%, based on starting alcohol.

### Fifth consecutive run:

Eight pieces, 7.32 grams lithium added to reactor, mix heated to 62.9°C, 78.35 grams alcohol added, reaction heated to reflux. Conversions at 20. 40, 60, and 79 min were 39, 63, 87, and 96%. Product decanted and 421 ml THF added to reactor.

### Sixth consecutive run:

No further lithium metal added to this run. The mixture from the fifth consecutive run was heated to 55.9°C and 76.21 grams of t-butyl alcohol added [1.03 moles] all at once. The mixture was heated to reflux. Conversions at 20, 60, 80, and 180 min were 16, 28, 69, and 96%, resp. Product decanted and 7.8 grams of alcohol added to cloudy solution. The remaining metal in the flask was covered with 421 ml of THF. The next day the solution in the bottle was clear.

### Seventh consecutive run:

No further metal was added to this run. There should be only enough metal to react with another charge of alcohol. The reactor contents were heated to 62°C and 75.48 grams of alcohol added and then the reaction heated to reflux. Conversions at 60, 120, 180, 240, 270, 300 and 334 min were 35, 60, 83, 94.5, 95.7, 96.6 and 96.6%, resp.

Note 1.: It was found on larger [pilot plant] runs where overall reaction times were somewhat slower due to a greater proportionate size of the bulk metal employed [see Table I] that the product solutions generated from consecutive 3X metal runs were essentially clear at the end of the reactions and did not require any further addition of tert-alcohol and overnight standing to effect clarification.

Note 2.: The lithium metal used in these runs contained 0.0035% sodium.

### Comparative Single Run Employing Bulk Lithium [Table III]

In this run, only a single equivalent of lithium metal in the form of cubes is used instead of three equivalents as in consecutive runs 3-5 above. The object of the experiment is to determine the relative overall reaction times and also to compare the overall reaction time of this run with the seventh consecutive run above where only one equivalent of metal is used.

A solution of 84.57 grams of tert-butyl alcohol [1.141 moles] and 378 ml of tetrahydrofuran were heated to reflux and nine pieces [7.2 grams, 1.0375 g. eq] of lithium metal in the form of 1x1x1 cm cubes added as quickly as possible. The mixture was heated and stirred at reflux and samples removed periodically to determine conversion. At 60, 180, 300, 420 and 480 min conversions were 12, 35, 68, 91, and 97%, respectively.

Both the 3X equivalents run and the seventh consecutive run [above] were considerably faster than the comparative single run.

### Comparative Runs Employing Lithium Dispersions [Table II]

### Exemplary Run--The Preparation of Lithium t-Butoxide Using Low Sodium Containing Lithium Dispersion

A weight of 27.7 grams of a 30 weight percent lithium dispersion, wherein the lithium metal contained 0.0148% sodium, was washed three times with dry hexane and once with dry pentane and then was blown dry with argon gas. The dry metal powder weighed 8.86 grams [1.276 moles]. The metal was transferred to a one liter three necked flask equipped as described above with the aid of most of a charge of 525 ml of dry THF and heated to reflux. A weight of 95.4 grams of tert-butyl alcohol was diluted with the remaining THF [ca 50 ml] and slowly added to the stirred contents of the flask from a dropping funnel over a 46 min period. No agglomeration of the metal was noted during the reaction. Samples for analysis were withdrawn through a syringe filter at 30, 60, and 100 minutes. Conversions were 93.7, 98.8, and 99.7%, respectively. A cloudy, white solution was obtained.

Another, similar run was made, using a lithium dispersion made from lithium metal containing less than 0.01% sodium [# 8599]. Conversions after 15 and 135 min were 93 and 96%. The product solution required 2.5 hours to filter completely [slow filtration] compared to just several minutes for one of the bulk metal laboratory runs described above.

### Comparative Run Using a High Sodium Containing Lithium Dispersion

A weight of 23.1 grams of a 30 weight percent lithium dispersion in mineral oil containing 6.93 grams [0.9986 gr. eq.] of lithium metal possessing a sodium content of 0.76% was transferred with the aid of 361 ml of dry THF to a one liter three necked flask equipped as above and heated to reflux. A solution of 81.4 grams [1.10 moles] of tert-butyl alcohol and 50 ml of dry THF was added to the contents of the flask over a 42 min period at reflux. The lithium metal particles agglomerated into bigger pieces. Samples of the product were filtered through a syringe filter and analyzed for total alkalinity. Conversions were 86.6 % [30 min], 90% [55 min], 92.3% [115 min], 92.5% [235 min], and 95.1% [330 min].

### Exemplary Run--The Preparation of Lithium t-Butoxide Employing Bulk Lithium and a Catalyst

A weight of 81.3 grams [1.096 moles] of tert-butyl alcohol and 3.0 grams [0.05 moles] of isopropyl alcohol were mixed with 410 ml of tetrahydrofuran and heated to reflux [see apparatus description above].

A weight of 6.99 grams [1.007 moles] of lithium metal rods, 1.27 cm in diameter and one centimeter long was added to the refluxing solution all at once. The sodium content of the lithium metal was 0.0093%. The reaction was allowed to proceed for 30 minutes and a sample taken for total alkalinity in order to calculate the degree of conversion [39%]. Subsequent samples were taken at 60, 90, 120, 150 and 190 minutes, at which point there was no longer any hydrogen release noted. The degree of conversion for these last five samples were 68, 79, 89, 93, and 96%.

A number of runs are catalogued in Table V using different catalysts as well as a comparative run with no catalyst.

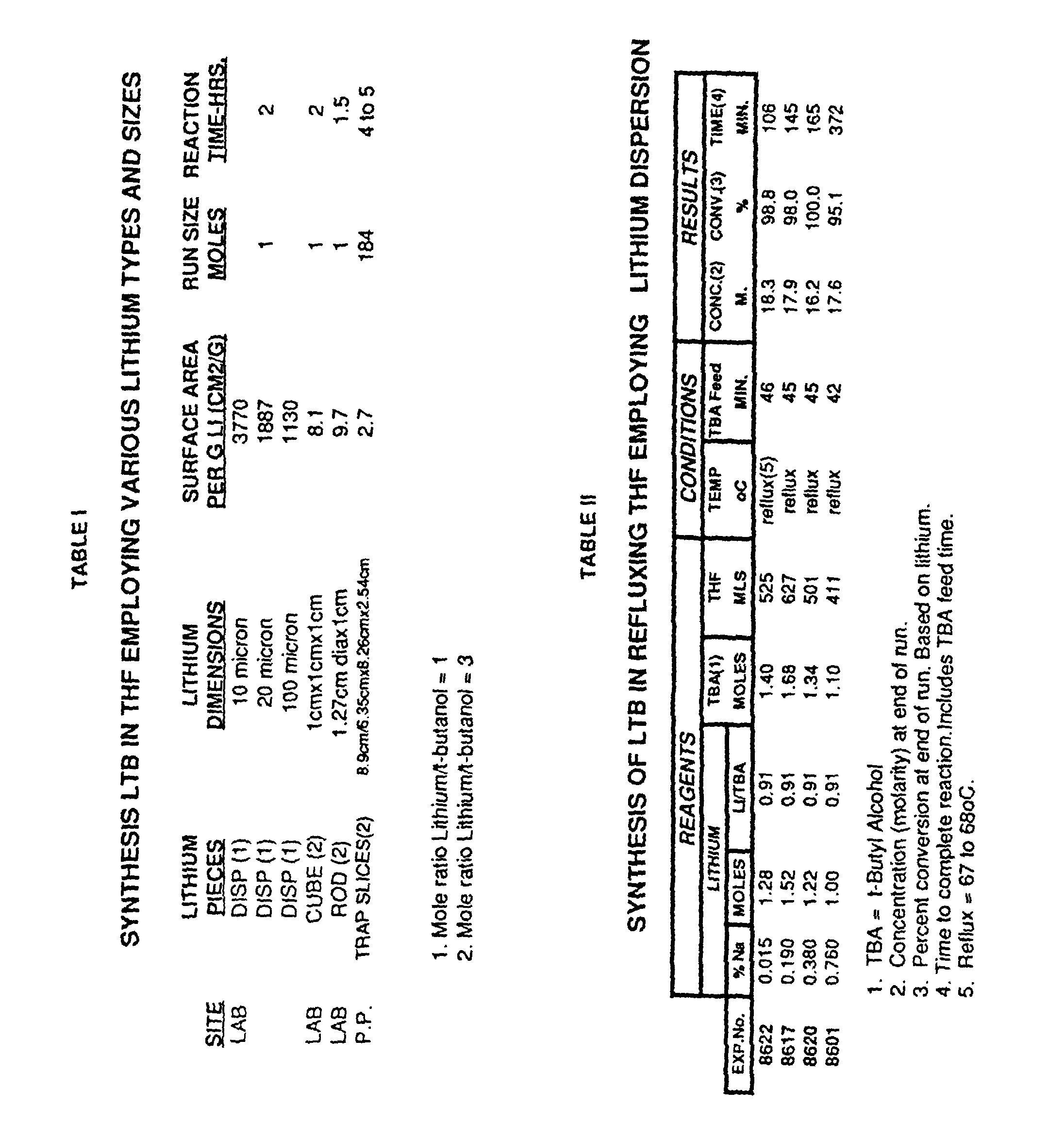

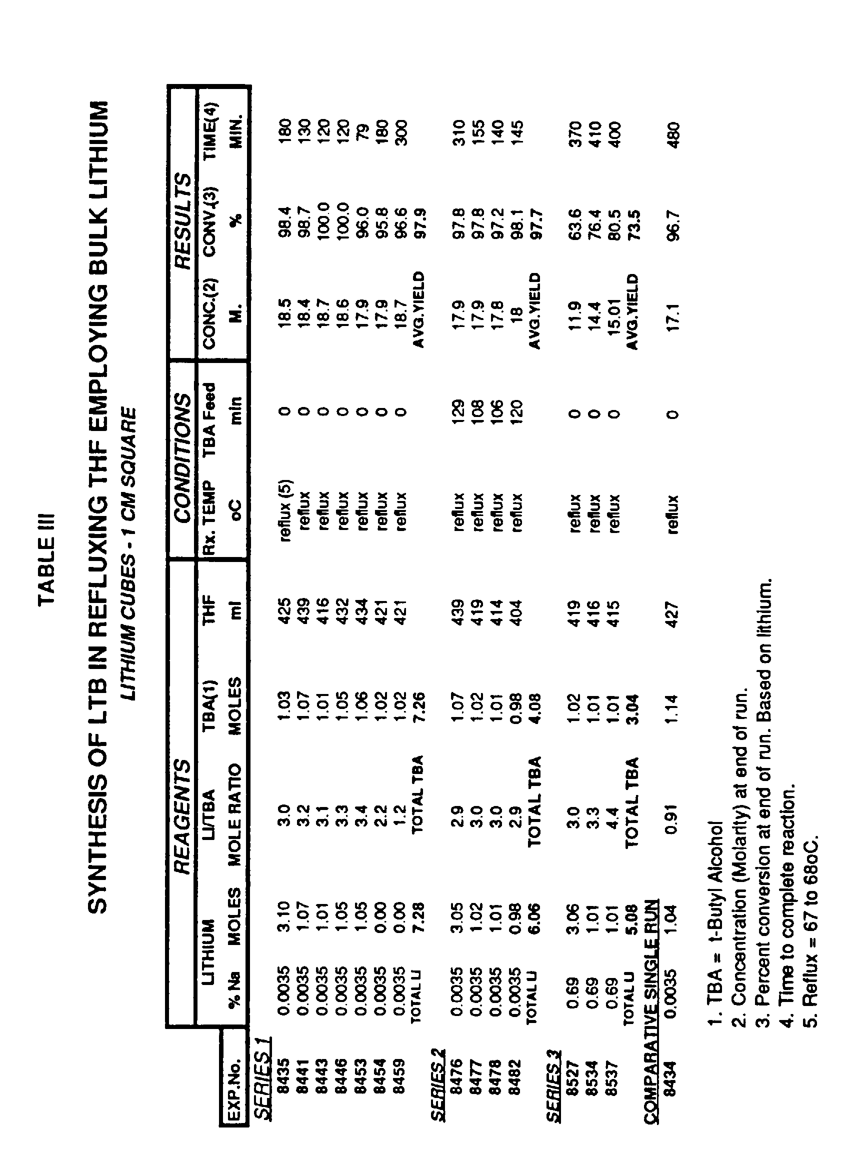

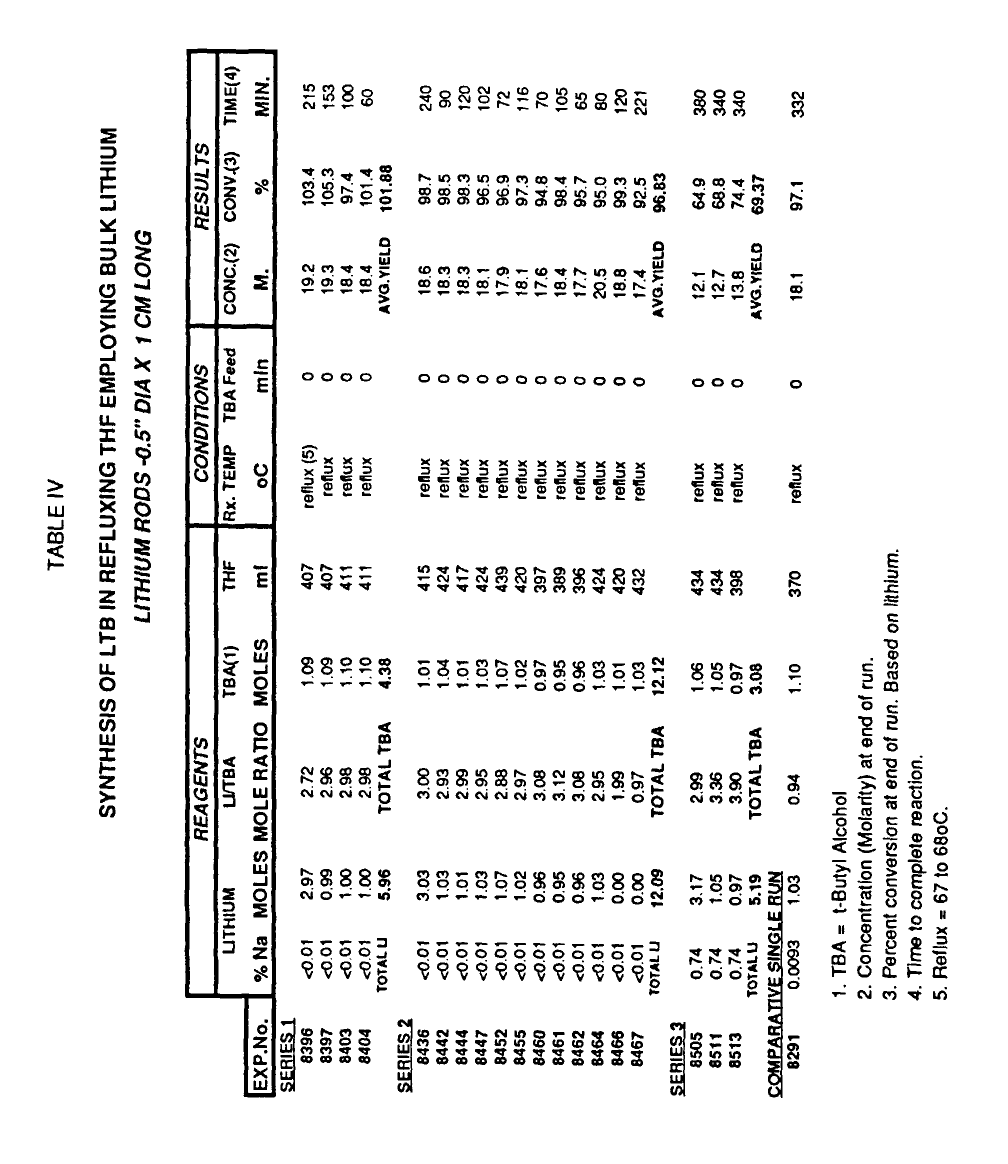

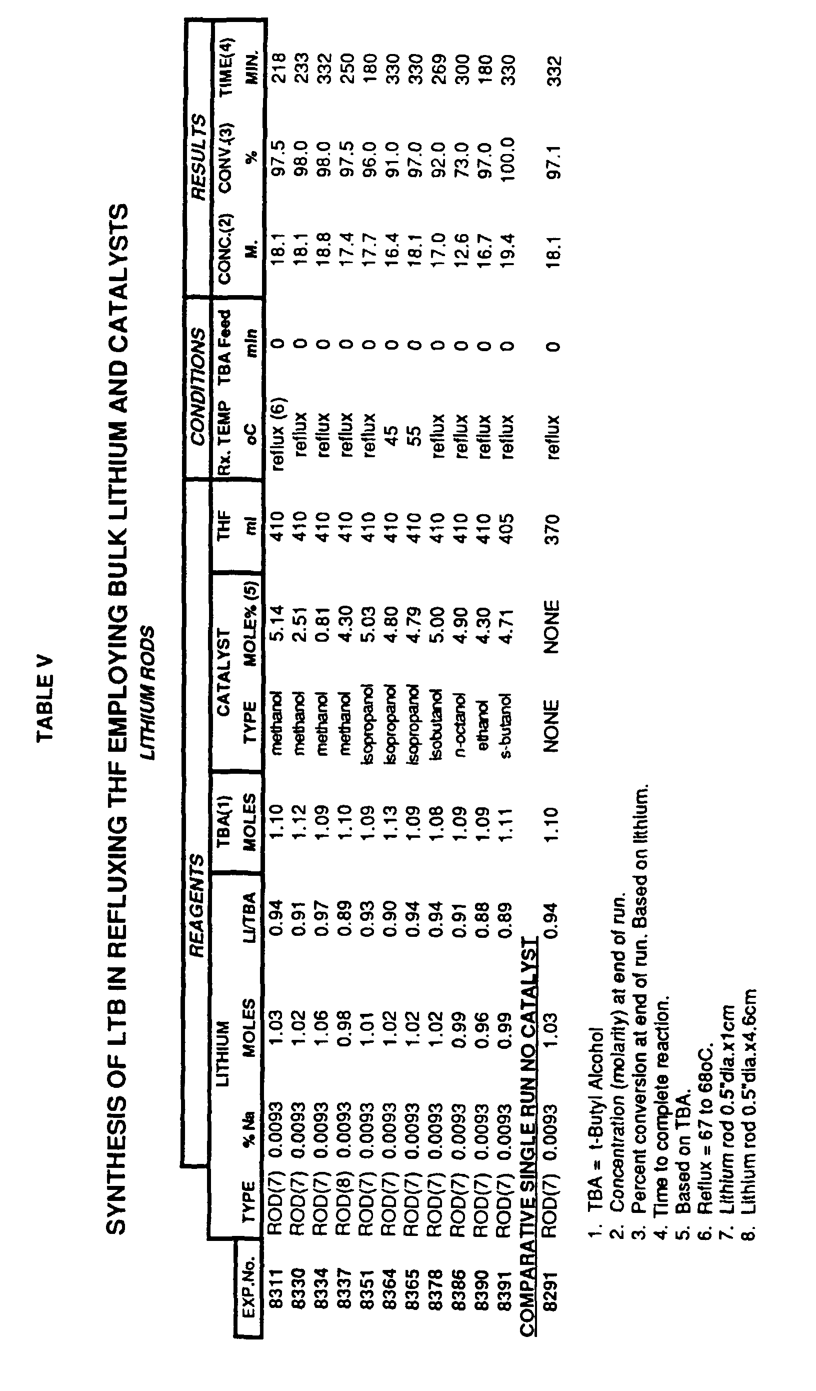

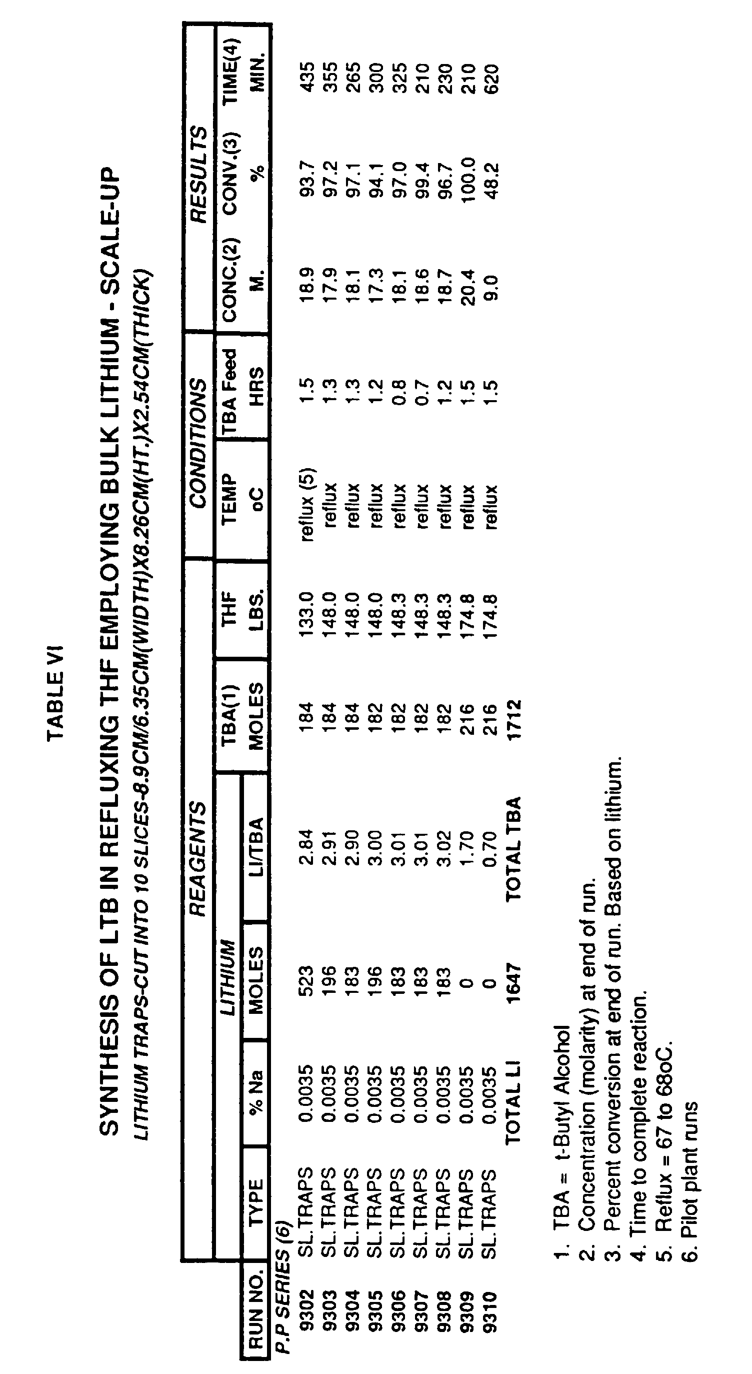

## Claims

1. A process for preparing a solution of a lithium tertiary-alkoxide characterized by the steps of reacting, in a reaction vessel, lithium bulk metal pieces of a weight greater than 0.5 grams per piece, containing less than 0.6% by weight of sodium, with a tertiary alkyl alcohol containing 3 to 10 carbon atoms, in mole ratios of metal to alcohol ranging from 1 to 1 to 10 to 1 in a solvent selected from ethereal or hydrocarbon solvents under an inert atmosphere at an elevated temperature between 34.6°C and 100°C for 1 to 10 hours, cooling the product lithium tertiary-alkoxide and separating the product lithium tertiary-alkoxide solution from the unreacted lithium metal in the reaction vessel.

2. The process of claim 1 characterized in that the tertiary alcohol is tertiary-butyl alcohol the solvent is tetrahydrofuran and the lithium metal to tertiary alcohol ratio is 3 to 1.

3. The process of claim 1 or 2 characterized in that the sodium content of the lithium metal is less than 0.01 weight percent.

4. A process as claimed in any of the preceding claims, wherein the lithium bulk metal pieces are cubes, rods or trapezoid slices.

5. A process as claimed in claim 4 wherein the cubes are 1 cm x 1 cm x 1 cm.

6. A process as claimed in claim 4 wherein the rods are 1.27 cm (dia 1 x 1 cm).

7. A process as claimed in claim 4 wherein the trapezoid slices are 8.9/6.35 cm x 8.26 cm x 2.54 cm.

8. The process of any of the preceding claims characterized in that the elevated temperature is the boiling point of tetrahydrofuran.

9. The process of any of the preceding claims characterized in that the separation of the product solution is carried out by decantation.

10. The process of any of the preceding claims for preparing of lithium tert-alkoxides, further characterized by adding 2-10 mole percent of C₁-C₃ alcohol catalyst to the reaction of lithium metal and a tertiary alcohol in an ethereal or hydrocarbon medium.

11. The process of claim 10 characterized in that tert-alkoxide is lithium tert-butoxide, the C₁-C₃ alcohol catalyst is methyl alcohol and the solvent is tetrahydrofuran.

12. The process of any of the preceding claims in which additional solvent and sufficient lithium metal or alcohol are added to the reaction vessel to re-establish the mole ratio of lithium metal to alcohol thereby causing the formation of further lithium tertiary alkoxide, separating the product lithium tertiary alkoxide solution from the unreacted lithium metal in the reaction vessel and repeating this a number of times.

13. The process of claim 12 characterised in that the number of repetitions of said reaction steps is at least three.

14. The process of claim 12 or 13 characterized in that the solvent added in the repeated steps is added immediately after removal of the lithium tertiary alkoxide solution.

15. A solution of lithium tert-alkoxide and a ethereal or hydrocarbon solvent or a mixture thereof, wherein the sodium content is less than 0.6 percent by weight in the lithium metal.

16. A solution of lithium tert-alkoxide as claimed in claim 15, wherein said solution has a sodium content of from 0.001 to 0.01 percent by weight in the lithium metal.

17. A solution of lithium tert-alkoxide and an ethereal or hydrocarbon solvent or a mixture thereof, said solution having a sodium content of less than 0.6 percent by weight in the lithium metal and comprising a C1-C3 alcohol.

## Patentansprüche

1. Verfahren zur Herstellung einer Lösung aus einem Lithium-tert.-alkoxid, gekennzeichnet durch die Schritte des Reagierens, in einem Reaktionsgefäß, von großen Lithiummetallstücken mit einem Gewicht höher als 0,5 Gramm pro Stück, das weniger als 0,6 Gew.-% Natrium enthält, mit einem tertiären Alkohol, der 3 bis 10 Kohlenstoffatome enthält, in Molverhältnissen von Metall zu Alkohol, die sich im Bereich von 1 zu 1 bis 10 zu 1 bewegen, in einem Lösungsmittel, das aus Ether- oder Kohlenwasserstofflösungsmitteln ausgewählt wird, unter einer inerten Atmosphäre bei einer erhöhten Temperatur zwischen 34,6°C und 100°C für eine Zeitdauer von 1 bis 10 Stunden, Abkühlen des Produktes Lithium-tert.-alkoxid und Trennen des Produktes Lithium-tert.-alkoxid-Lösung von dem nicht in Reaktion getretenen Lithiummetall in dem Reaktionsgefäß.

2. Verfahren nach Anspruch 1, dadurch gekennzeichnet, daß der tertiäre Alkohol tertiärer Butylalkohol ist, das Lösungsmittel Tetrahydrofuran ist und das Verhältnis von Lithiummetall zu tertiärem Alkohol 3 zu 1 ist.

3. Verfahren nach Anspruch 1 oder 2, dadurch gekennzeichnet, daß der Natriumgehalt des Lithiummetalls weniger als 0,01 Gew.-% beträgt.

4. Verfahren nach einem der vorangehenden Ansprüche, worin die großen Lithiummetallstücke Würfel, Stäbe oder trapezförmige Scheiben sind.

5. Verfahren nach Anspruch 4, worin die Würfel 1 cm x 1 cm x 1 cm sind.

6. Verfahren nach Anspruch 4, worin die Stäbe 1,27 cm (Durchmesser 1 x 1 cm) sind.

7. Verfahren nach Anspruch 4, worin die trapezförmigen Scheiben 8,9/6,35 cm x 8,26 cm x 2,54 cm sind.

8. Verfahren nach einem der vorangehenden Ansprüche dadurch gekennzeichnet, daß die erhöhte Temperatur der Siedepunkt von Tetrahydrofuran ist.

9. Verfahren nach einem der vorangehenden Ansprüche, dadurch gekennzeichnet, daß die Trennung der Produkt-Lösung durch Dekantierung durchgeführt wird.

10. Verfahren nach einem der vorangehenden Ansprüche zum Herstellen von Lithium-tert.-alkoxiden, darüber hinaus gekennzeichnet durch Zufügen von 2 - 10 Mol-% C₁-C₃-Alkoholkatalysator zu der Reaktion von Lithiummetall und einem tertiären Alkohol in einem Ether- oder Kohlenwasserstoffmedium.

11. Verfahren nach Anspruch 10, dadurch gekennzeichnet, daß tert.-Alkoxid Lithium-tert.-butoxid ist, der C₁-C₃-Alkoholkatalysator Methylalkohol ist und das Lösungsmittel Tetrahydrofuran ist.

12. Verfahren nach einem der vorangehenden Ansprüche, worin dem Reaktionsgefäß zusätzliches Lösungsmittel und ausreichend Lithiummetall oder Alkohol zugefügt werden, um das Molverhältnis von Lithiummetall zu Alkohol wiederherzustellen, wodurch die Bildung von weiterem Lithium-tert.-Alkoxid veranlaßt wird, Trennen des Produktes Lithium-tert.-alkoxid-Lösung von dem nicht in Reaktion getretenen Lithiummetall in dem Reaktionsgefäß und mehrmaliges Wiederholen dieses Schrittes.

13. Verfahren nach Anspruch 12, dadurch gekennzeichnet, daß die Anzahl von Wiederholungen genannter Reaktionsschritte mindestens bei drei liegt.

14. Verfahren nach Anspruch 12 oder 13, dadurch gekennzeichnet, daß das Lösungsmittel in den wiederholten Schritten sofort nach Entfernung der Lithium-tert.-alkoxid-Lösung zugefügt wird.

15. Lösung von Lithium-tert.-alkoxid und einem Ether- oder Kohlenwasserstofflösungsmittel oder einem Gemisch davon, worin der Natriumgehalt weniger als 0,6 Gew.-% in dem Lithiummetall beträgt.

16. Lösung aus Lithium-tert.-alkoxid nach Anspruch 15, worin genannte Lösung einen Natriumgehalt von 0,001 bis 0,01 Gew.-% in dem Lithiummetall aufweist.

17. Lösung aus Lithium-tert.-alkoxid und einem Ether- oder Kohlenwasserstofflösungsmittel oder einem Gemisch davon, wobei genannte Lösung einen Natriumgehalt von weniger als 0,6 Gew.-% in dem Lithiummetall aufweist und einen C₁-C₃-Alkohol umfaßt.

## Revendications

1. Procédé pour préparer une solution d'un alkoxyde tertiaire de lithium, caractérisé par les étapes consistant à faire réagir, dans un vase de réaction, des morceaux de métal de lithium en vrac d'un poids supérieur à 0,5 gramme par morceau, contenant moins de 0,6% en poids de sodium, avec un alcool alkylique tertiaire contenant de 3 à 10 atomes de carbone, selon des rapports molaires de métal à alcool allant de 1 à 1 à 10 à 1 dans un solvant sélectionné à partir de solvants éthérés ou d'hydrocarbures dans une atmosphère inerte à une température élevée d'entre 34,6°C et 100°C pendant 1 à 10 heures, refroidir l'alkoxyde tertiaire de lithium produit et séparer la solution d'alkoxyde tertiaire de lithium produit du métal de lithium inaltéré dans le vase de réaction.

2. Procédé de la revendication 1 caractérisé en ce que l'alcool tertiaire est de l'alcool tertiaire butylique le solvant est du tétrahydrofurane et le rapport de métal de lithium à alcool tertiaire est de 3 à 1.

3. Procédé de la revendication 1 ou 2 caractérisé en ce que la teneur en sodium du métal de lithium est de moins de 0,01 pour cent en poids.

4. Procédé tel que revendiqué dans l'une quelconque des revendications précédentes, dans lequel les morceaux de métal de lithium en vrac sont des cubes, des barres ou des tranches trapézoïdales.

5. Procédé tel que revendiqué dans la revendication 4 dans lequel les cubes font 1 cm x 1 cm x 1 cm.

6. Procédé tel que revendiqué dans la revendication 4 dans lequel les barres font 1,27 cm (1 x 1 cm de diamètre).

7. Procédé tel que revendiqué dans la revendication 4 dans lequel les tranches trapézoïdales font 8,916,35 cm x 8,26 cm x 2,54 cm.

8. Procédé de l'une quelconque des revendications précédentes, caractérisé en ce que la température élevée est le point d'ébullition du tétrahydrofurane.

9. Procédé de l'une quelconque des revendications précédentes caractérisé en ce que la séparation de la solution de produit est réalisée par décantation.

10. Procédé de l'une quelconque des revendications précédentes pour la préparation d'alkoxydes tertiaires de lithium, caractérisé en outre par l'ajout de 2-10 moles pour cent d'alcool C₁-C₃ catalyseur à la réaction du métal de lithium et d'un alcool tertiaire dans un milieu éthéré ou d'hydrocarbures.

11. Procédé de la revendication 10 caractérisé en ce que l'alkoxyde tertiaire est du butoxyde tertiaire de lithium, l'alcool C₁-C₃ catalyseur est de l'alcool méthylique et le solvant est du tétrahydrofurane.

12. Procédé de l'une quelconque des revendications précédentes dans lequel du solvant additionnel et suffisamment de métal de lithium ou d'alcool sont ajoutés dans le vase de réaction pour établir de nouveau le rapport molaire de métal de lithium à alcool causant ainsi la formation d'alkoxyde tertiaire de lithium supplémentaire, séparer la solution d'alkoxyde tertiaire de lithium produit du métal de lithium inaltéré dans le vase de réaction et répéter ceci plusieurs fois.

13. Procédé de la revendication 12 dans lequel le nombre de répétitions desdites étapes de réaction est d'au moins trois.

14. Procédé de la revendication 12 ou 13 caractérisé en ce que le solvant ajouté aux étapes répétées est ajouté immédiatement après l'enlèvement de la solution d'alkoxyde tertiaire de lithium.

15. Solution d'alkoxyde tertiaire de lithium et d'un solvant éthéré ou d'hydrocarbures ou d'un mélange de ceux-ci, dans laquelle la teneur en sodium est de moins de 0,6 pour cent en poids dans le métal de lithium.

16. Solution d'alkoxyde tertiaire de lithium telle que revendiquée dans la revendication 15, dans laquelle ladite solution a une teneur en sodium de 0,001 à 0,01 pour cent en poids dans le métal de lithium.

17. Solution d'alkoxyde tertiaire de lithium et d'un solvant éthéré ou d'hydrocarbures ou d'un mélange de ceux-ci, ladite solution ayant une teneur en sodium de moins de 0,6 pour cent en poids dans le métal de lithium et comprenant un alcool C₁-C₃.
